# EUROPÄISCHE PATENTSCHRIFT

(11) **EP 1 259 193 B1**
(45) Veröffentlichungstag und Bekanntmachung des Hinweises auf die Patenterteilung: **17.11.2010**
(21) Anmeldenummer: 01916903.6
(22) Anmeldetag: 28.02.2001
(51) Int. Cl.: A61F 2/24

(54) **VERANKERUNG FÜR IMPLANTIERBARE HERZKLAPPENPROTHESEN**
ANCHORING SYSTEM FOR IMPLANTABLE HEART VALVE PROSTHESES
SYSTEME D'ANCRAGE POUR PROTHESES DE VALVULES CARDIAQUES IMPLANTABLES

(30) Priorität: 28.02.2000 DE 10010073
(43) Veröffentlichungstag der Anmeldung: 27.11.2002
(73) Patentinhaber: JenaValve Technology Inc., Wilmington, DE 19801 (US)
(72) Erfinder: FERRARI, Markus, 07747 Jena (DE); LANG, Klaus, 34576 Homberg/Efze (DE); FIGULLA, Hans-Reiner, 07749 Jena (DE); DAMM, Christoph, 07743 Jena (DE); WEBER, Carsten, 07743 Jena (DE); RISSE, Stefan, 07749 Jena (DE); GUYENOT, Volker, 07749 Jena (DE); MOHAUPT, Matthias, 07778 Altengönna (DE); HARNISCH, Gerd, 07749 Jena (DE); DÖRRER, Peggy, 37083 Göttingen (DE)
(74) Vertreter: Trinks, Ole
(86) Internationale Anmeldenummer: PCT/DE2001/000836
(87) Internationale Veröffentlichungsnummer: WO 2001/064137

(56) Entgegenhaltungen:
- EP-A- 0 896 813
- WO-A-00/18333
- US-A- 4 485 816
- US-A- 5 234 447
- US-A- 5 817 113
- US-A- 5 891 160

## Beschreibung

Die Erfindung betrifft eine Verankerung für Herzklappenprothesen, die ohne größeren operativen Eingriff, bei dem auf herkömmliche Art und Weise eine vollständige Öffnung des Thorax erforderlich war, durch Einführen über die Aorta implantierbar ist.

In der Vergangenheit wurden die verschiedensten Versuche unternommen und auch Lösungen angeboten, mit denen Herzklappenprothesen durch die Aorta bis hin zum Herzen geführt und nach einem Entfaltungs- oder Aufspannprozess dort fest implantiert werden sollten.

So ist u.a. in US 5,855,601 ein sogenannter Stent beschrieben, an dem eine Herzklappenprothese befestigt werden kann. Der Stent wird im Wesentlichen aus drahtförmigem Formgedächtnismetall gebildet, das in einer Gitterstruktur, ein zylinderförmiges Gebilde darstellt. Das Formgedächtnismetall kann bei Temperaturen unter 37 °C zusammengefaltet werden und soll im zusammengefalteten Zustand mittels eines Katheters über die Aorta bis zum Herzen geführt, dort nach Abschalten einer Kühlung bei Erreichen der Temperatur von ca. 37 °C die eingeprägte Form annehmen und in dem entsprechenden entfalteten Zustand an der Aorteninnenwand anliegen.

Infolge der Grenzen des Formgedächtnismetalles kann ein solcher Stent jedoch nur schwer soweit miniaturisiert und zusammengefaltet werden, um problemlos durch die Aorta geführt zu werden und außerdem im entfalteten Zustand so dimensioniert zu sein, dass er dann an der Aorteninnenwand anliegt und auch sicher gehalten wird.

Außerdem bereitet die Herstellung einer so aufgebauten und konstruierten Verankerungsstütze aus herkömmlichem und im erforderlichen Temperaturbereich geeigneten Formgedächtnismetall (z.B. Nitinol) Probleme und ist mit einem entsprechend hohen Fertigungsaufwand verbunden, da ein solcher Körper in der Regel aus einem Vollmaterial durch Laserstrahlschneiden herausgeschnitten wird.

Im Stand der Technik unter Art. 54(3) EPÜ werden hakenförmige Elemente aus ELGILOY offenbart.

Es ist daher Aufgabe der Erfindung, eine Möglichkeit vorzuschlagen, mit der Herzklappenprothesen, über die Aorta zum Herzen geführt und dort fixiert werden können.

Erfindungsgemäß wird diese Aufgabe mit den Merkmalen des Anspruchs 1 gelöst. Vorteilhafte Ausgestaltungsformen und Weiterbildungen der Erfindung ergeben sich mit den in den untergeordneten Ansprüchen genannten Merkmalen.

Erfindungsgemäß werden unmittelbar an den Kommissuren einer biologischen Herzklappenprothese, die z.B. von einem Schweineherz entnommen sein kann, oder einer ansonsten künstlichen Herzklappenprothese hakenförmige Elemente befestigt, deren Haken durch das Herzklappenprothesenmaterial nach außen geführt sind und im implantierten Zustand in die Aortenwand eingestochen sind, bzw. die Aortenwand durchstechen, so dass die Herzklappenprothese an der Aortenwand sicher befestigt werden kann.

Außerdem können zusätzliche hakenförmige Elemente auch am Außenring der Herzklappenprothese befestigt sein.

Dabei können die hakenförmigen Elemente an die Herzklappenprothese angenäht sein.

Einfacher und mit geringerem Aufwand verbunden, ist es aber die hakenförmigen Elemente von innen in die Herzklappenprothese einzuführen und zumindest die Haken durch das Herzklappenprothesenmaterial zu stoßen, wobei es zweckmäßig ist, die hakenförmigen Elemente auf einem Steg oder mit einem Basiselement auszubilden, wodurch gesichert wird, dass lediglich die Haken, der hakenförmigen Elemente aus der Herzklappenprothese herausragen und der übrige Teil durch eine entsprechende Größe den Halt im Inneren der Herzklappenprothese sichert.

So kann ein Steg beispielsweise in Form eines flachen Stabes bzw. einer länglichen Platte ausgebildet sein, an dem mindestens zwei hakenförmige Elemente ausgebildet sind.

Ein Basiselement kann beispielsweise plattenförmig in Kreisform ausgebildet sein, wobei die Querschnittsfläche des Kreises, ein Durchstoßen des Herzklappenprothesenmaterials verhindern kann.

Die hakenförmigen Elemente, die an Stegen bzw. auf Basiselementen angeordnet sind, können im Wesentlichen stiftförmig ausgebildet sein. Dabei ist/sind an dem stiftförmigen Teil ein oder mehrere Widerhaken zur Verankerung in der Aortenwand ausgebildet, die sich nach dem Einpressen in oder hinter der Aortenwand verhaken.

Dabei kann die Widerhakenform einem Formgedächtnismetall eingeprägt werden, so dass die Widerhaken erst oberhalb der Sprungtemperaturen nach außen gerichtet abstehen und so das Einstechen und Durchstoßen der Aortenwand erleichtert ist.

Werden Stege verwendet, an denen die hakenförmigen Elemente ausgebildet oder an diesen befestigt sind, können diese an den Kommissuren einer Herzklappenprothese so befestigt werden, dass sie im implantierten Zustand in Längsrichtung der Aorta ausgerichtet sind.

Es kann aber auch besonders vorteilhaft sein, die Stege spiralförmig auszubilden, so dass sie durch die Spiralform an die Innenkontur der Aorta angepasst sind und nach der Verankerung mittels der Haken an dieser unmittelbar anliegen, wobei infolge der Spiralform ein größerer Umfangsbereich der Aorteninnenwand abgedeckt und demzufolge ein erhöhter Dichtgrad erreicht werden kann.

Werden einzelne ansonsten voneinander getrennte hakenförmige Elemente mit Basiselementen verwendet, sollten diese ebenfalls über die Kommissuren verteilt angeordnet sein, wobei ebenfalls eine der Spiralform folgende Anordnung oder eine Anordnung zweier gegenläufig gewundener Spiralen möglich ist.

Werden Stege mit hakenförmigen Elementen verwendet, sollten diese Stege eine Länge aufweisen, die zumindest so groß, wie die Länge der Kommissuren der Herzklappenprothese sein.

Die an den verschiedenen Kommissuren befestigten Stege sind untereinander nicht verbunden.

Für das Einführen der Herzklappenprothese mit der daran befestigten Verankerung ist es außerdem günstig, wenn zumindest die hakenförmigen Elemente aus einem Formgedächtnismetall bestehen, das sichert, dass die hakenförmigen Elemente während der Einführphase flach anliegen und nur einen geringen Einführwiderstand und ein geringes Volumen erreicht werden und erst am Implantationsort, nach Abstellen der Kühlung, die gewünschte Form und ein Aufrichten der Haken für die Verankerung in der Aortenwand erfolgt.

Die effektive Höhe der hakenförmigen Elemente sollte dann mindestens 2 mm betragen, so dass sie in dieser Höhe aus der Herzklappenprothese herausragen und in die Aortenwand eindringen bzw. diese durchstechen.

Vorteilhaft ist es außerdem, die Haken der verschiedenen hakenförmigen Elemente in unterschiedlichen Richtungen abzuwinkeln, so dass ebenfalls den Bedingungen in der Aorta, in der Nähe des Herzens vorkommenden Druck- und Strömungsbedingungen Rechnung tragend, eine sichere Fixierung der erfindungsgemäßen Verankerung erreicht werden kann.

Vorteilhaft können benachbarte hakenförmige Elemente aufeinander zu gerichtet abgewinkelt sein, bzw. eine solche Stellung bei Verwendung von Formgedächtnismetall eingeprägt werden. Dies führt bei Erreichen bzw. Überschreiten der Sprungtemperatur zu einer Verklammerung benachbarter hakenförmiger Elemente in der Aortenwand.

Außerdem kann im Inneren der Herzklappenprothese, im Bereich deren Außenringes ein zusätzliches ringförmiges Element befestigt sein, das in der Phase des Einführens durch die Aorta ebenfalls zusammengedrückt werden kann, und ein kleineres Volumen in Anspruch nimmt. Hierfür kann ein elastisches Material, wie z.B. ein geeigneter Kunststoff bzw. Gummi, gegebenenfalls mit einer Spiralfeder versehen, oder auch eine Spiralfeder allein oder ein Formgedächtnismetall verwendet werden.

Die mit der erfindungsgemäßen Verankerung versehene Herzklappenprothese kann mittels eines Katheders, an dem eine entsprechende Mechanik ausgebildet ist, in die Aorta eingeführt werden, wobei die Mechanik, das Entfalten der Herzklappenprothese und das Lösen der in der gewünschten Position platzierten Herzklappenprothese sichern sollte.

Es kann aber auch ein Ballonkatheter eingesetzt werden, wobei die mit der erfindungsgemäßen Verankerung versehene Herzklappenprothese aufgestülpt und in dieser Position in die Aorta eingeführt werden kann. Wird dann der Implantationsort erreicht, wird der Ballon mit Druck beaufschlagt und die Herzklappenprothese mit den hakenförmigen Elementen gegen die Aortenwand gepresst und die Haken können dann in der Aortenwand verankert werden, wobei bei der Verwendung von Formgedächtnismetall das Ballonkatheter auch für die erforderliche Kühlung verwendet werden kann.

Die Oberfläche des Ballons eines solchen Katheters kann entsprechend konturiert sein, so dass z.B. die Basiselemente bzw, die Stege infolge der Elastizität des Ballons und der entsprechend ausgebildeten Kontur die Stege bzw. die Basiselemente in der Durchführungsphase durch die Aorta sicher hält und bei der Aufweitung des Ballons dieser Halt gelöst werden kann.

Es kann aber auch ein Katheter mit einem sich selbsttätig entfaltenden Mechanismus verwendet werden, der wiederum ebenfalls aus einem Formgedächtnismetall besteht, verwendet werden, um die Befestigung der Herzklappenprothese mit der erfindungsgemäßen Verankerung zu gewährleisten.

In jedem Fall ist es jedoch zweckmäßig, in der Durchführungsphase die Herzklappenprothese zu umhüllen, um unerwünschte Verletzungen der Aortenwand vor Erreichen des Implantationsortes zu vermeiden und außerdem eine entsprechende Wärmeisolierung, bei erforderlicher Kühlung zu sichern. Nach Erreichen des Implantationsortes kann diese Umhüllung aufgerissen bzw. ein Entriegelungsmechanismus aktiviert und mit dem Katheter wieder aus der Aorta entfernt werden.

Wird ein Entfaltungsmechanismus aus einem Formgedächtnismetall verwendet, kann dieser, wie bereits erwähnt, als ringförmiges Element ausgebildet werden, das im Bereich des Außenringes der Herzklappenprothese an dieser befestigt ist, Verwendung finden. Ein solches ringförmiges Element kann dann die Stabilität der implantierten Herzklappenprothese und eine erhöhte Abdichtfunktion sichern.

Nachfolgend soll die Erfindung anhand eines Ausführungsbeispieles näher erläutert werden.

Dabei zeigt:

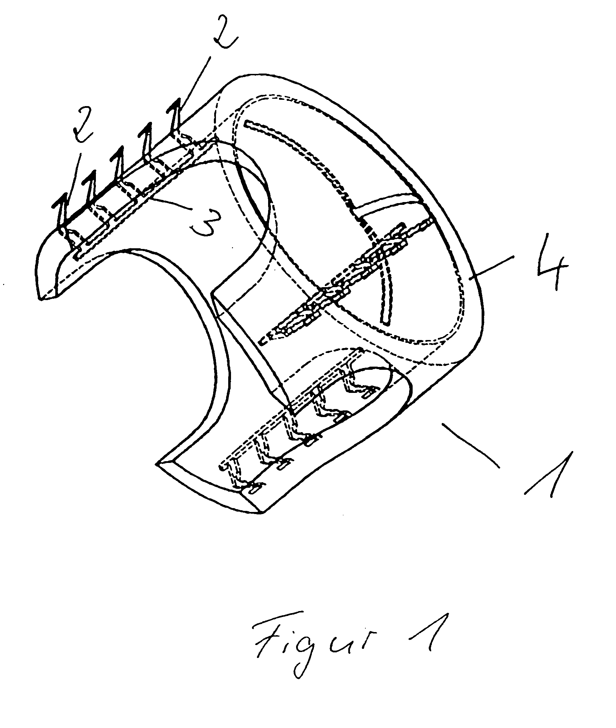

- Figur 1: ein Beispiel einer erfindungsgemäßen Ver- ankerung an einer Herzklappenprothese.

Bei dem in der Figur 1 gezeigten Beispiel einer erfindungsgemäßen Verankerung für eine Herzklappenprothese 1 sind an Stegen 3 hakenförmige Elemente 2 ausgebildet und jeweils ein solcher Steg 3 an einer Kommissur der Herzklappenprothese 1 angeordnet und dort befestigt.

Bei diesem Beispiel bestehen sowohl die Stege 3, wie auch die hakenförmigen Elemente 2 aus einem Formgedächtnismetall, wie dies z.B. unter der Bezeichnung Nitinol erhältlich ist. Für die Befestigung werden die Stege 3 von innen in die Herzklappenprothese 1 eingeführt und einfach durch die Kommissuren der Herzklappenprothese 1 gepresst, so dass diese durchstochen wird.

Dem verwendeten Formgedächtnismetall, kann die in der Figur 1 gezeigte Form eingeprägt werden, die nach Überschreiten der Sprungtemperatur von 37 °C eingenommen wird. In der Einführphase der Herzklappenprothese 1 sollten die hakenförmigen Elemente 2 jedoch möglichst um nahezu 90° in Richtung auf die Längsachse der Stege 3 geneigt sein, so dass sie ein relativ kleines Volumen erfordern und nur geringfügig überstehen, so dass das Einführen erleichtert und Verletzungen der Aortenwand vermieden werden können.

Bei dem hier gezeigten Beispiel sind auf einem Steg 3 jeweils fünf hakenförmige Elemente ausgebildet. Es sollten insgesamt jedoch mindestens fünfzehn hakenförmige Elemente verwendet werden.

Die Anzahl der hakenförmigen Elemente 2 kann aber ohne weiteres variiert werden und es besteht außerdem die Möglichkeit, mehrere solcher Stege 3, mit hakenförmigen Elementen 2 an einer Kommissur der Herzklappenprothese 1 zu befestigen.

Dabei können die Stege 3, nicht wie hier gezeigt, in einem Winkel geneigt ausgerichtet sein, wobei zwei solcher Stege 3 auch entgegengesetzt zueinander geneigt sein können.

In nicht dargestellter Form kann ein zusätzliches ringförmiges Element im Bereich des Außenringes 4 der Herzklappenprothese 1, an dieser befestigt sein.

Außerdem können zur Verbesserung der Fixierung an den hakenförmigen Elementen 2 bzw. deren Haken auch Widerhaken ausgebildet sein.

## Patentansprüche

1. Implantierbare Herzklappenprothese mit einer Verankerung, wobei die Verankerung mehrere hakenförmige Elemente (2) oder hakenförmige Elemente (2) mit Widerhaken und mindestens ein Basiselement oder Steg (3) aufweist, mit welchem die hakenförmigen Elemente (2) oder die hakenförmigen Elemente (2) mit Widerhaken gehalten werden, wobei die hakenförmigen Elemente (2) oder die hakenförmigen Elemente (2) mit Widerhaken an mindestens einer Kommissur der Herzklappenprothese (1) befestigt sind, wobei die hakenförmigen Elemente (2) durch das Herzklappenmaterial nach außen geführt sind, und wobei die hakenförmigen Elemente (2) oder die hakenförmigen Elemente (2) mit Widerhaken aus einem Formgedächtnismetall bestehen und ausgelegt sind, während der Einführphase bei der Implantation der Herzklappenprothese (1) flach anzuliegen und sich bei Erreichen bzw. Überschreiten der für das Formgedächtnismetall charakteristischen Sprungtemperatur aufzurichten.

2. Herzklappenprothese nach Anspruch 1, wobei die hakenförmigen Elemente (2) an die Herzklappenprothese (1) angenäht sind.

3. Herzklappenprothese nach Anspruch 1 oder 2, wobei mindestens zwei hakenförmigen Elemente (2) auf einem Basiselement oder Steg (3) angeordnet sind.

4. Herzklappenprothese nach Anspruch 1 oder 2, wobei mehrere Basiselemente oder Stege (3) in Längsrichtung der Herzklappenprothese ausgerichtet sind.

5. Herzklappenprothese nach Anspruch 1, wobei die hakenförmigen Elemente (2) im entfalteten Zustand, zur Verankerung in der Aortenwand, eine effektive Höhe von mindestens 2 mm haben, mit der sie aus der Herzklappenprothese herausragen.

6. Herzklappenprothese nach Anspruch 1, wobei die hakenförmigen Elemente (2) stiftförmig ausgebildet und mit Widerhaken versehen ausgebildet sind.

7. Herzklappenprothese nach Anspruch 1, wobei die Widerhaken der hakenförmigen Elemente (2) in verschiedene Richtungen abgewinkelt sind.

8. Herzklappenprothese nach Anspruch 1, wobei die hakenförmigen Elemente (2) im Inneren der Herzklappenprothese (1) mit mindestens einem Basiselement gehalten sind.

9. Herzklappenprothese nach Anspruch 3, wobei mehrere Basiselemente in Längsrichtung der Herzklappenprothese ausgerichtet sind.

10. Herzklappenprothese nach Anspruch 3, wobei mehrere Basiselemente spiralförmig ausgebildet sind.

11. Herzklappenprothese nach Anspruch 3, wobei mehrere Basiselemente mindestens so lang wie die Kommissuren sind.

12. Herzklappenprothese nach einem der vorhergehenden Ansprüche, wobei im Inneren der Herzklappenprothese (1) im Bereich des Außenringes ein ringförmiges Element befestigt ist.

13. Herzklappenprothese nach Anspruch 12, wobei das ringförmige Element aus einem elastischen Material oder Formgedächtnismetall besteht.

## Claims

1. Implantable heart valve prosthesis comprising an anchoring system, wherein the anchoring system includes several hook-shaped elements (2) or hook-shaped elements (2) with barbs and at least one base element or web (3) by means of which the hook-shaped elements (2) or the hook-shaped elements (2) with barbs are held, wherein the hook-shaped elements (2) or the hook-shaped elements (2) with barbs are secured to at least one commissure of the heart valve prosthesis (1), wherein the hook-shaped elements (2) are guided in an outward direction through the heart valve material, and wherein the hook-shaped elements (2) or the hook-shaped elements (2) with barbs are made of a shape memory metal and are adapted to lie flat during the introduction phase when the heart valve prosthesis (1) is implanted and right themselves once the transition temperature characteristic for the shape memory metal is reached or exceeded.

2. Heart valve prosthesis according to claim 1, wherein the hook-shaped elements (2) are sewn to the heart valve prosthesis (1).

3. Heart valve prosthesis according to claim 1 or 2, wherein at least two hook-shaped elements (2) are arranged on a base element or web (3).

4. Heart valve prosthesis according to claim 1 or 2, wherein several base elements or webs (3) are oriented in the longitudinal direction of the heart valve prosthesis.

5. Heart valve prosthesis according to claim 1, wherein the hook-shaped elements (2) have an effective height of at least 2 mm in a deployed condition, at which they protrude from the heart valve prosthesis for being anchored in the aorta wall.

6. Heart valve prosthesis according to claim 1, wherein the hook-shaped elements (2) are pin-shaped and provided with barbs.

7. Heart valve prosthesis according to claim 1, wherein the barbs of the hook-shaped elements (2) are bent in various directions.

8. Heart valve prosthesis according to claim 1, wherein the hook-shaped elements (2) inside the heart valve prosthesis (1) are held by at least one base element.

9. Heart valve prosthesis according to claim 3, wherein several base elements are oriented in the longitudinal direction of the heart valve prosthesis.

10. Heart valve prosthesis according to claim 3, wherein several base elements are designed in a helical manner.

11. Heart valve prosthesis according to claim 3, wherein several base elements are at least as long as the commissures.

12. Heart valve prosthesis according to one of the preceding claims, wherein an annular element is secured inside the heart valve prosthesis (1) in the area of the outer ring.

13. Heart valve prosthesis according to claim 12, wherein the annular element is made of an elastic material or shape memory metal.

## Revendications

1. Prothèse de valvule cardiaque implantable comprenant un ancrage, ledit ancrage comprenant plusieurs éléments en forme de crochet (2) ou plusieurs éléments en forme de crochet avec ardillon (2), et au moins un élément de base ou barrette (3) au moyen duquel les éléments en forme de crochet (2) ou éléments en forme de crochet avec ardillon (2) sont maintenus, et les éléments en forme de crochet (2) ou éléments en forme de crochet avec ardillon (2) sont fixés au niveau d'au moins une commissure de la prothèse de valvule cardiaque (1), lesdits éléments en forme de crochet (2) étant menés vers l'extérieur à travers le matériau de valvule cardiaque, et dans laquelle les éléments en forme de crochet (2) ou éléments en forme de crochet avec ardillon (2) sont réalisés en un métal à mémoire de forme et conçus de manière à se coucher à plat pendant la phase d'introduction lors de l'implantation de la prothèse de valvule cardiaque (1) et à se redresser lorsqu'ils ont atteint ou dépassé la température de transition caractéristique pour le métal à mémoire de forme.

2. Prothèse de valvule cardiaque selon la revendication 1, dans laquelle les éléments en forme de crochet (2) sont cousus sur la prothèse de valvule cardiaque (1).

3. Prothèse de valvule cardiaque selon la revendication 1 ou 2, dans laquelle au moins deux éléments en forme de crochet (2) sont agencés sur un élément de base ou barrette (3).

4. Prothèse de valvule cardiaque selon la revendication 1 ou 2, dans laquelle plusieurs éléments de base ou barrettes (3) sont orientés en direction longitudinale de la prothèse de valvule cardiaque.

5. Prothèse de valvule cardiaque selon la revendication 1, dans laquelle les éléments en forme de crochet (2) présentent, dans l'état déployé en vue de l'ancrage dans la paroi de l'aorte, une hauteur effective d'au moins 2 mm, par laquelle ils dépassent hors de la prothèse de valvule cardiaque.

6. Prothèse de valvule cardiaque selon la revendication 1, dans laquelle les éléments en forme de crochet (2) sont réalisés sous forme de tige et sont dotés d'ardillons.

7. Prothèse de valvule cardiaque selon la revendication 1, dans laquelle les ardillons des éléments en forme de crochet (2) sont coudés dans différentes directions.

8. Prothèse de valvule cardiaque selon la revendication 1, dans laquelle les éléments en forme de crochet (2) sont maintenus à l'intérieur de la prothèse de valvule cardiaque (1) avec au moins un élément de base.

9. Prothèse de valvule cardiaque selon la revendication 3, dans laquelle plusieurs éléments de base sont orientés en direction longitudinale de la prothèse de valvule cardiaque.

10. Prothèse de valvule cardiaque selon la revendication 3, dans laquelle plusieurs éléments de base sont réalisés sous forme spiralée.

11. Prothèse de valvule cardiaque selon la revendication 3, dans laquelle plusieurs éléments de base sont au moins aussi longs que les commissures.

12. Prothèse de valvule cardiaque selon l'une des revendications précédentes, dans laquelle un élément de forme annulaire est fixé à l'intérieur de la prothèse de valvule cardiaque (1) dans la région de la bague extérieure.

13. Prothèse de valvule cardiaque selon la revendication 12, dans laquelle l'élément de forme annulaire est réalisé en un matériau élastique ou en métal à mémoire de forme.
